**Europäisches Patentamt**

(19) **European Patent Office**

**Office européen des brevets**

(11) Veröffentlichungsnummer: **0 056 951 B1**

(12) # EUROPÄISCHE PATENTSCHRIFT

(45) Veröffentlichungstag der Patentschrift:
19.06.85

(51) Int. Cl.⁴: **C 07 K 7/40,** C 12 P 21/02

(21) Anmeldenummer: **82100217.7**

(22) Anmeldetag: **14.01.82**

(54) **Verfahren zur Herstellung von Humaninsulin oder dessen Derivaten aus Schweineinsulin oder dessen Derivaten.**

(30) Priorität: **17.01.81 DE 3101382**

(43) Veröffentlichungstag der Anmeldung:
**04.08.82 Patentblatt 82/31**

(45) Bekanntmachung des Hinweises auf die Patenterteilung:
**19.06.85 Patentblatt 85/25**

(84) Benannte Vertragsstaaten:
**AT BE CH DE FR GB IT LI NL SE**

(56) Entgegenhaltungen:
**EP - A - 0 017 938**
**EP - A - 0 045 187**
**DE - A - 2 460 753**
**GB - A - 2 069 502**
**US - A - 3 276 961**
**US - A - 3 903 068**

**CHEMICAL ABSTRACTS, Band 92, Nr. 17, 1980, Seite 245, Nr. 142751u, Columbus, Ohio, USA K. MORIHARA et al.: "Achromobacter protease I-catalyzed conversion of porcine insulin into human insulin"**
**NATURE, Band 280, Nr. 5721, August 1979, Selten 412,413, Basingstoke, G.B. K. MORIHARA et al: "Semi-Synthesis of human insulin by trypsin-catalysed replacement of Ala-B30 by Thr in porcine insulin"**
**Biochem. Biophys. Res. Comm. 92 (1980) 396-402;**

(73) Patentinhaber: **HOECHST AKTIENGESELLSCHAFT,**
**Postfach 80 03 20, D-6230 Frankfurt am Main 80 (DE)**

(72) Erfinder: **Obermeier, Rainer, Dr., Langenhainer Weg 14,**
**D-6234 Hattersheim am Main (DE)**
Erfinder: **Ludwig, Jürgen, Ringstrasse 13,**
**D-6486 Brachttal (DE)**
Erfinder: **Selpke, Gerhard, Dr., Brunnenweg 4,**
**D-6200 Wiesbaden (DE)**

(56) Entgegenhaltungen: (Fortsetzung)
**LEHNINGER: "Blochemle, 2. Auflage, Verlag Chemie, S. 158**
**Biochem. I. 211 (1983) 671-676**
**Hoppe Seylers Z. Physiol. Chem. 362 (1981) 1591-98.**

## Beschreibung

Menschliches Insulin und Insulin vom Schwein unterscheiden sich durch die carboxylständige Aminosäure in Position B 30 der Insulin-B-Kette. Bei Humaninsulin folgt dem Lysyl-Rest in B 29 ein Threonin, bei Schweineinsulin Alanin.

Außer der Totalsynthese von Humaninsulin (1) Märkli et al. Hoppe Seyler's Z. Physiol. Chem. 360 1699—1632 (1979) ermöglichen verschiedene semisynthetische Verfahren den Austausch von Alanin gegen Threonin im Schweineinsulin als Ausgangsmaterial.

Zur Herstellung größerer Humaninsulin-Mengen ist die Totalsynthese zu aufwendig.

Bei den Seminsynthese-Verfahren von Ruttenberg (2) US-Pat. 3 903 068 sowie Obermeier und Geiger (3) R. Obermeier et al. Hoppe—Seylers's Z. Physiol. Chem. 357 759—767 (1976) wird ein durch tryptische Verdauung hergestelltes Desoktapeptid-B 23-30-Insulin vom Schwein mittels peptidchemischer Methoden mit einem geschützten, synthetischen Oktapeptid der Humaninsulinsequenz B 23-30 verknüpft. Nach Abspaltung aller Schutzgruppen folgen aufwendige Reinigungsschritte. Die Ausbeuten an Humaninsulin sind klein.

Höhere Ausbeuten bei der Umwandlung von natürlichen Schweineinsulin in Humaninsulin lassen sich mit enzymatischen Verfahren erzielen.

Inouye et al. J. Amer. Chem. Soc. 101 751—752 (1979) (4) haben ein Verfahren entwickelt, bei dem Desoktapeptid-B 23-30-Insulin vom Schwein mit synthetischem Oktapeptid-B 23-30 (human) unter tryptischer Katalyse zu Humaninsulin umgesetzt wird. Nachteil dieser Reaktion ist die Verwendung eines synthetischen Oktapeptids, das, wie bei Ruttenberg und Obermeier, mit erheblichem Aufwand hergestellt werden muß.

Ökonomischer ist die Umwandlung, wenn nur die letzte Aminosäure-B 30 des Schweineinsulins ausgetauscht wird. Im US-Pat. 3 276 961 wird von Bodanszky et al. ein Verfahren beschrieben, mit dem aus tierischen Insulinen mit Hilfe von Enzymen wie Trypsin und Carboxypeptidase A in Gegenwart von Threonin Humaninsulin hergestellt wird. Das Verfahren ist jedoch nicht durchführbar, da unter den dort beschriebenen Bedingungen nicht nur Lys-Ala (B 29-30) sondern auch andere Peptidbindungen im Insulin gespalten werden.

H. G. Gattner et al. in: Insulin ed. D. Brandenburg, A. Wollmer 1980 Proc. 2nd Intern. Insulin Symposium 1979, S. 118—123 (5) und K. Morihara et al.: Nature 280 412—413 (1979) (6) und EP-A-0 017 938 (6a) gehen in einem zweistufigen Verfahren von Des-Ala B 30 Insulin (Schwein) aus, und verknüpfen dieses mit Hilfe von Trypsin mit Threonin-methylester oder Threonin-tert.-butylester zu dem entsprechenden Humaninsulinester. Nach Abspaltung der Estergruppe durch Behandlung mit Natronlauge bzw. Trifluoressigsäure wird Humaninsulin in guter Ausbeute gewonnen.

Die beiden letztgenannten bekannten Verfahren verwenden Des-Ala-B 30-Insulin vom Schwein als Ausgangsmaterial. Dieses wird mit Hilfe von Carboxypeptidase A (CPA) aus Schweineinsulin gewonnen. CPA spaltet schrittweise carboxylständige neutrale und saure L-Aminosäuren von Peptiden und Proteinen. Die Aminosäuren werden mit unterschiedlicher Spaltungskinetik abgespalten. Der enzymatische Abbau stoppt an basischen Aminosäuren z. B. am Lysin-B 29 oder am Arginin-B 22 der Insulin-B-Kette. Damit läßt sich der Alaninrest in Position B 30 der Insulin-B-Kette ohne weiterschreitenden Kettenabbau eliminieren.

Der Nachteil der CPA-Verdauung liegt jedoch im gleichzeitigen Angriff des Enzyms an der C-terminalen Aminosäure Asparagin in Position A 21 der A-Kette des Insulins. Bei den üblichen Verdauungsbedingungen für CPA werden auf Grund der verschiedenen Spaltungsgeschwindigkeiten etwa 10—20% des Asparagins und gleichzeitig 80—90% des Alanins vom Schweineinsulin eliminiert. Ein derartiges Verdauungsprodukt enthält demnach neben unumgesetztem Insulin ein Gemisch von Des-Ala-B 30-des-Asn-A 21-, Des-Ala-B 30- und Des-Asn-A 21-Insulin.

E. W. Schmitt et al. Hoppe Seyler's Z. Physiol. Chem. 359 799—802 (1978) (8) ist es gelungen, durch Verwendung $NH_4^+$-haltiger Puffer, die Entstehung von Des-Ala-B 30-des-Asn-A 21-Insulin auf weniger als 5—10% zu reduzieren. Trotz säulenchromatographischer Reinigung läßt sich aber nicht ausschließen, daß ein so hergestelltes Humaninsulin noch deutliche immunologische Reaktionen auslöst.

»Aus Biochem. Biophys. Res. Commun. 92 [1980] 396—402 ist ein zweistufiges Verfahren zur Herstellung von Humaninsulin ausgehend von Schweineinsulin bekannt. In der ersten Stufe dieses Verfahrens wird Schweineinsulin in einem $NH_4HCO_3$-Puffer vom pH 8,3 mit Hilfe von Achromobacter-Protease I in Des-Ala-B 30-Schweineinsulin überführt, welches dann in der zweiten Verfahrensstufe in Gegenwart der gleichen Protease mit L-Threonin-tert.-butylester unter Bildung von Humaninsulin-B 30-tert.-butylester gekuppelt wird. Das Kupplungsmedium ist ein Gemisch aus Tris-Puffer vom pH 6,5 und organischen Lösungsmitteln. Nach saurer Abspaltung der Estergruppe wird Humaninsulin in 52%iger Ausbeute gewonnen.«

Es wurde nun ein Semisynthese-Verfahren gefunden, mit dem unter Umgehung der CPA-Verdauung mit Hilfe von Trypsin in einem einzigen Schritt die Umwandlung von Schweineinsulin in Humaninsulinester gelingt. Aus dem Ester kann auf üblichem Wege Humaninsulin dargestellt werden. Die Gesamtausbeute bei der einstufigen Reaktion beträgt 50—65%. Neben der wesentlich einfacheren Reaktionsführung, liegt der Vorteil dieses Verfahrens in der Gewinnung vom Humaninsulin, das keine der obengenannten Verunreinigungen mehr enthalten kann und somit auch zum Einsatz bei immunologischen Problemfällen geeignet ist.

»Die Erfindung betrifft somit ein Verfahren zur Herstellung von Humaninsulin oder seinen Derivaten aus Schweineinsulin oder dessen Derivaten, das dadurch gekennzeichnet ist, daß man Schweineinsulin bzw. ein Derivat des Schweineinsulins bei einem pH-Wert unterhalb seines isoelektrischen Punktes in Gegenwart von Trypsin oder einem trypsinähnlichen Ferment mit einem Überschuß eines Threoninesters oder eines seiner Derivate mit freier Aminogruppe umsetzt und gewünschtenfalls danach die Schutzgruppe(n) abspaltet.«

Als Ausgangsmaterial für die erfindungsgemäße Umsetzung eignen sich außer dem unveränderten Schweineinsulin dessen Derivate, wie sie durch Einbau von Schutzgruppen an freiliegenden Funktionen oder durch Abspaltung oder Austausch einzelner Aminosäuren erhalten werden können. Als Verfahrensprodukt resultiert im Fall der Verwendung von derartigen Derivaten des Schweineinsulins ein Humaninsulin, das die entsprechenden Schutzgruppen oder Sequenzen enthält. Ein bevorzugtes Derivat des Schweineinsulins, das der erfindungsgemäßen Reaktion unterworfen werden kann, ist das Des-Phe$^{B}$1-Schweineinsulin, das bei der erfindungsgemäßen Umsetzung zum entsprechenden Des-Phe$^{B}$1-Humaninsulin führt.

Weiterhin sind bevorzugte Derivate des Schweineinsulins solche, die in der Position N$^{\alpha B1}$ eine Schutzgruppe tragen. Bevorzugte Schutzgruppen in dieser Position sind insbesondere der t-Butyloxycarbonyl-(BOC-) oder der Dimethoxy-phenylpropyloxycarbonyl(DDZ-)-Rest. Weitere Schutzgruppen sind bekannt aus E. Wünsch, Methoden der organischen Chemie (Houben—Weyl) Band XV/1, Stuttgart 1974.

Die Umsetzung wird erfindungsgemäß bei einem pH-Wert unterhalb des isolelektrischen Punktes des verwendeten Ausgangs-Insulins bzw. Derivates durchgeführt. Im Falle von Schweineinsulin liegt der isoelektrische Punkt bei pH 5,4. Demgemäß empfiehlt sich bei der Verwendung von Schweineinsulin als Ausgangsmaterial ein Arbeiten bei einem pH-Wert unterhalb 5,4. Dem Arbeiten bei niedrigen pH-Werten sind durch die Beständigkeit des Insulins und die Enzymaktivität in stark saurem Milieu Grenzen gesetzt. Empfehlenswert ist, die Reaktion in einem pH-Bereich zwischen 4 und dem isoelektrischen Punkt auszuführen.

Als zweckmäßig hat es sich erwiesen, das Insulin bzw. Insulinderivat mit einem Threonin-Esteracetat in einem wäßrigen Medium vorzunehmen, das vorzugsweise einen Wassergehalt ≤50 Volumen-% aufweist und das mit einer schwachen organischen Säure, vorzugsweise Essigsäure, auf einen schwach sauren pH-Wert von etwa pH 5 eingestellt wurde. Der Vorteil dieser speziellen Verfahrensvariante liegt in erster Linie in einer gegenüber dem Arbeiten in wäßrigorganischen Lösungsmitteln höheren Ausbeute.

Weitere Vorteile sind die Einsparung des organischen Lösungsmittels und eine erleichterte Aufarbeitung des Reaktionsgemisches, da das organische Lösungsmittel nicht abgetrennt werden braucht.

Die Umsetzung kann bei Raumtemperatur durchgeführt werden. Zur Beschleunigung empfiehlt sich leichtes Erwärmen, jedoch sollte eine Temperatur von etwa 40°C nicht überschritten werden. Ein Arbeiten unter Kühlung bringt keine Vorteile.

Neben Trypsin eignen sich für das erfindungsgemäße Verfahren auch solche Fermente, die aus der Literatur als trypsinähnlich bekannt sind, d. h. solche, die spezifische Peptidbindungen am Carboxylende basischer Aminosäuren spalten. Die Menge des anzuwendenden Ferments spielt eine untergeordnete Rolle. Sie kann zwischen 1 : 1 und 100 : 1 liegen, wobei sich die Zahlen auf das Gewichtsverhältnis Schweineinsulin : Ferment beziehen. Vorteilhaft arbeitet man bei einem Gewichtsverhältnis von etwa 10 : 1.

Als Threoninester können alle bekannten L-Threoninester verwendet werden. Beispiele geeigneter L-Threoninester sind L-Threonin-tert.-butylester, L-Threonin-O.-tert.-butyl-tert.-butylester und L-Threoninmethylester. Unter Derivaten von Threoninestern im Sinne der Erfindung sind solche zu verstehen, die an der OH-Funktion des Threonins eine Schutzgruppe, insbesondere die Äther-Schutzgruppe tragen.

Der Threoninester ist gegenüber dem Insulin im Überschuß einzusetzen. Der Überschuß soll die etwa 10- bis 100fache molare Menge des Insulins betragen.

Die erfindungsgemäße Umsetzung führt zunächst zu B 30-Estern des Humaninsulins, die gewünschtenfalls nach an sich bekannten Verfahren durch Spaltung der Estergruppe in das freie Humaninsulin überführt werden können.

Vorteilhaft ist es, den Ester vor seiner Überführung in das freie Insulin den erforderlichen Reinigungsoperationen beispielsweise mit Hilfe bekannter säulenchromatographischer Methoden zu unterziehen.

Das erhaltene Insulin kann in üblichen Darreichungsformen zubereitet als Arzneimittel zur Behandlung des Diabetes mellitus verwendet werden.

## Ausführungsbeispiele

### Beispiel 1

120 mg Insulin vom Schwein und 231 mg Thr-(OBu$^t$)Bu$^t$ werden in 2 ml 0,1molarem Pyridinacetat-Puffer von pH 4,0 suspendiert und durch Zugabe von 3 ml DMF gelöst. Der pH-Wert der Lösung wird überprüft und gegebenenfalls erneut mit Essigsäure oder Pyridin auf pH 4,0 eingestellt. Zur klaren Lösung werden bei 30°C 5 mg TPCK-Trypsin gegeben. Im Abstand von 4 Stunden werden 2 weitere Portionen von 5 mg TPCK-Trypsin zugegeben. Das Reaktionsmedium wird dann noch 16 Stunden bei 35°C gerührt. Danach wird die Lösung mit 1 n HCl auf pH 2—3 angesäuert und durch Zugabe von Äthanol (1 ml) und Äther (5 ml) gefällt. Der Niederschlag wird abzentrifugiert und mit Äther verrieben. Der pulverförmige Rückstand wird durch Verteilungschromatographie an Sephadex® LH20 wie bei Obermeier et al. beschrieben gereinigt. Die den Humaninsulinester enthaltenden Fraktionen aus der Trennung werden im Vakuum bei Raumtemperatur eingeengt und mit Aceton/Äther gefällt. Zurückgewonnenes nicht umgesetztes Schweineinsulin kann erneut für die Semisynthese eingesetzt werden. Der entstehende Niederschlag wird abzentrifugiert und getrocknet. Ausbeute: 81 mg. Dieses Produkt wird zur Abspaltung der Schutzgruppen in 3 ml 6 n mit Anisol gesättigter HCl unter kurzem Erwärmen auf 40°C gelöst. Danach wird gekühlt und bei 0°C mit 10%iger Natronlauge auf pH 2—3 eingestellt.

Durch Zugabe von 30 ml Aceton wird das Humaninsulin gefällt. Der Niederschlag wird abzentrifugiert und im Vakuum getrocknet. Ausbeute 77 mg Humaninsulin.

Das so gewonnene Humaninsulin zeigt im Blutzuckersenkungstest am Kaninchen volle biologische Aktivität von 26 I. E./mg bezogen auf Schweineinsulin.

Die Aminosäureanalyse entspricht den theoretischen Werten für Humaninsulin:

|  | theoretisch | gefunden |
|---|---|---|
| Glu | 7 | 7,00 |
| Ala | 1 | 1,04 |
| Thr | 3 | 2,88 |
| Lys | 1 | 1,01 |

### Beispiel 2

120 mg Insulin vom Schwein und 133 mg Threoninmethylester werden wie im Beispiel 1 zu Humaninsulinmethylester umgesetzt und gereinigt. Das so gewonnene Produkt (73 mg) wird mit 0,1 ml 0,1 n NaOH 15 Minuten bei 0°C gerührt. Danach wird mit 0,1 n HCl (0,5 ml) neutralisiert, dialysiert und gefriergetrocknet. Das entstandene Humaninsulin (68 mg) ist biologisch voll aktiv und die Aminosäureanalyse entspricht den theoretischen Werten.

### Beispiel 3

120 mg Insulin vom Schwein und 175 mg Threonin-tert.-butylester werden wie im Beispiel 1 zur Reaktion gebracht und gereinigt. Ausbeute 76 mg Humaninsulin-tert.-butylester. Das Produkt wird in 1 ml Trifluoressigsäure und 0,05 ml Anisol gelöst und bei Raumtemperatur 60 Minuten gerührt. Das so entstandene ungeschützte Humaninsulin wird durch Zugabe von 8 ml Äther gefällt und abzentrifugiert. Der Rückstand wird in Wasser gelöst, dialysiert und gefriergetrocknet. Ausbeute: 71 mg Humaninsulin mit voller biologischer Aktivität und korrekter Aminosäurezusammensetzung.

### Beispiel 4

115 mg Des-Phe$^{B}$1-Insulin vom Schwein werden mit 231 mg Thr(Bu$^t$)OBu$^t$ wie im Beispiel 1 umgesetzt und gereinigt. Ausbeute 75 mg Des-Phe$^{B}$1-Humaninsulin-B 30di-tert.-butylester.
Nach Abspaltung der tert.-Butylgruppen und Isolierung analog zu Beispiel 1 erhält man 70 mg freies Des-Phe$^{B}$1-Humaninsulin.
Biologische Aktivität: 26 I. E./mg
Aminosäureanalyse:

|  | theoretisch | gefunden |
|---|---|---|
| Glu | 7 | 7,00 |
| Ala | 1 | 0,98 |
| Thr | 3 | 2,90 |
| Phe | 2 | 2,10 |
| Lys | 1 | 1,03 |

### Beispiel 5

120 mg Insulin vom Schwein werden mit 231 mg Thr(Bu$^t$)OBu$^t$ wie im Beispiel 1 umgesetzt. Anstelle von Trypsin werden 0,50 ml Trypsin gebunden an Agarosegel verwendet. Nach Abfiltrieren der Trypsinagarose wird die Lösung wie in Beispiel 1 aufgearbeitet.
Ausbeute an Humaninsulin: 61 mg

### Beispiel 6

5 g Insulin vom Schwein und 30 g Thr(Bu$^t$)O-Bu$^t$-acetat werden in 20 ml $H_2O$ gelöst und mit Essigsäure der pH auf 5,0—5,2 eingestellt. Zur Lösung der Reaktionskomponenten werden 400 mg Trypsin gelöst in 2 ml $H_2O$ zugefügt. Der Verlauf der Reaktion wird mittels Acetatfolienelektrophorese überwacht. Bei maximaler Umsetzung (ca. 90%) des eingesetzten Schweineinsulins wird das rohe Reaktionsprodukt durch Zugabe von 200 ml Methanol und 50 ml Diisopropyläther gefällt. Nach Zentrifugation und Trocknung beträgt die Ausbeute 5,1 g Rohmaterial, das nach HPLC-analytischer Untersuchung 90% Humaninsulin-B 30-Bu$^t_2$ enthält.

## Beispiel 7

In 30 ml wäßriger 37,5%iger Essigsäure werden nacheinander 5 g $N^{\alpha B1}$-BOC-Schweineinsulin und 24 g Thr(Bu$^t$)OBu$^t$ gelöst und wie in Beispiel 6 mit 400 mg Trypsin in 2 ml H$_2$O versetzt. Die Reaktion verläuft wie in Beispiel 6 und wird nach maximaler Umsetzung durch Fällung mit Methanol/Diisopropyläther gestoppt. Nach Isolierung und Trocknung beträgt die Ausbeute 5,0 g Rohmaterial, das laut HPLC-Analytik 87% von $N^{B1}$-Boc-Humaninsulin-B 30-Bu$^t_2$ enthält.

## Beispiel 8

10 g Schweineinsulin werden in 45 ml Wasser unter Zugabe von 5 ml Essigsäure gelöst. Man setzt 33 g Thr(tBu)OtBu-acetat zu und gibt zu dieser klaren Lösung unter Rühren 0,8 g Trypsin, gelöst in 5 ml Wasser. Nach 16 Stunden bei Raumtemperatur wird die Reaktion durch Fällung mit 500 ml eines Gemisches aus Methanol und Diisopropylether (Volumenverhältnis 4 : 1) abgebrochen und wie im Beispiel 1 aufgearbeitet.
Ausbeute an Humaninsulinester: 4 g.

## Beispiel 9

10 g Schweineinsulin werden in 40 ml Wasser unter Zugabe von 5 ml Essigsäure zusammen mit 60 g Thr(tBu)OtBu-acetat gelöst. Zum Gemisch der Reaktionspartner werden 0,8 g Trypsin, in 2 ml Wasser vorgelöst, gegeben. Nach 16 Stunden bei Raumtemperatur wird wie im Beispiel 8 weiterverfahren und gereinigt.
Ausbeute nach Reinigung: 4,1 g Humaninsulinester.

## Beispiel 10

120 mg Schweineinsulin werden in 0,5 ml Wasser und 0,04 ml Essigsäure zusammen mit 380 mg Thr(tBu)OtBu-acetat gelöst. Zur Lösung werden 10 Einheiten Lysylendopeptidase-Lys-c1 (5 mg Protein) gegeben. Nach 16 h bei Raumtemperatur wird wie im Beispiel 8 weiterverfahren und gereinigt.
Ausbeute an Humaninsulinester: 61 mg.

## Beispiel 11

1,35 kg Thr(tBu)OtBu werden in 1 l Petrolether gelöst und mit Eis gekühlt. Zur Lösung werden 340 ml Essigsäure gerührt und die Lösung bis zu beendeter Kristallisation auf 0°C gekühlt. Die Kristallreste Thr(tBu)OtBu-acetat wird abfiltriert, mit Petrolether von 0°C gewaschen und im Vakuum getrocknet.
Ausbeute an Thr(tBu)OtBu-acetat: 1,44 kg (Fp 58−60°C).

## Patentansprüche

1. Verfahren zur Herstellung von Humaninsulin oder seinen Derivaten aus Schweineinsulin oder dessen Derivaten, dadurch gekennzeichnet, daß man Schweineinsulin bzw. ein Derivat des Schweineinsulins bei einem pH-Wert unterhalb seines isoelektrischen Punktes in Gegenwart von Trypsin oder einem trypsinähnlichen Ferment mit einem Überschuß eines Threoninesters oder eines seiner Derivate mit freier Aminogruppe umsetzt und gewünschtenfalls danach die Schutzgruppe(n) abspaltet.

2. Verfahren gemäß Anspruch 1, dadurch gekennzeichnet, daß die Reaktion in Abwesenheit eines organischen Lösungsmittels erfolgt, wobei vorzugsweise Schweineinsulin in einem Reaktionsmedium umgesetzt wird, dessen Wassergehalt $\geq 50$ Volumen-% ist.

3. Verfahren gemäß Anspruch 1 oder 2, dadurch gekennzeichnet, daß die Reaktion in Gegenwart einer schwachen organischen Säure erfolgt.

4. Verfahren gemäß einem oder mehreren der Ansprüche 1 bis 3, dadurch gekennzeichnet, daß die Reaktion in Gegenwart von Essigsäure erfolgt.

5. Verfahren gemäß einem oder mehreren der Ansprüche 1 bis 4, dadurch gekennzeichnet, daß bei einem pH-Wert zwischen 4 und dem isoelektrischen Punkt gearbeitet wird.

6. Verfahren gemäß Anspruch 2, dadurch gekennzeichnet, daß die Reaktion in Gegenwart einer schwachen organischen Säure, vorzugsweise Essigsäure erfolgt.

7. Verfahren gemäß einem oder mehreren der Ansprüche 1 bis 6, dadurch gekennzeichnet, daß Des-Phe$^{B1}$-Humaninsulin aus Des-Phe$^{B1}$-Schweineinsulin hergestellt wird.

8. Verfahren nach einem oder mehreren der Ansprüche 1 bis 6, dadurch gekennzeichnet, daß das Derivat des Schweineinsulins ein Schweineinsulin ist, das in $N^{\alpha B1}$-Stellung eine Schutzgruppe trägt.

9. Verfahren nach Anspruch 8, dadurch gekennzeichnet, daß die Schutzgruppe BOC oder DDZ ist.

10. Verfahren gemäß einem oder mehreren der Ansprüche 1 bis 9, dadurch gekennzeichnet, daß die Reaktion mit Thr(Bu$^t$)OBu$^t$ erfolgt.

## Claims

1. Process for the preparation of human insulin or its derivatives from pig insulin or its derivatives, characterized in that pig insulin or a derivative thereof is reacted at a pH-value below its isoectric point in the presence of trypsin or a trypsin-like ferment with an excess amount of a threonine ester or a derivative thereof having a free amino group, and that optionally the protective group(s) is split off.

2. Process according to claim 1, characterized in that the reaction is carried out in the absence of an organic solvent, preferably pig insulin being reacted in a reaction medium, the water content of which is equal or higher than 50% by volume.

3. Process according to claim 1 or 2, characterized in that the reaction is carried out in the presence of a weak organic acid.

4. Process according to one or more of claims 1 to 3, characterized in that the reaction is carried out in the presence of acetic acid.

5. Process according to one or more of claims 1 to 4, characterized in that there is operated at a pH-value between 4 and the isoelectric point.

6. Process according to claim 2, characterized in that the reaction is carried out in the presence of a weak organic acid, preferably acetic acid.

7. Process according to one or more of claims 1 to 6, characterized in that Des-Phe$^{B1}$ human insulin is prepared from Des-Phe$^{B1}$ pig insulin.

8. Process according to one or more of claims 1 to 6, characterized in that the derivative of the pig insulin is such pig insulin which carries a protective group in N$^{\alpha B1}$-position.

9. Process according to claim 8, characterized in that the protective group is BOC or DDZ.

10. Process according to one or more of claims 1 to 9, characterized in that the reaction is carried out with Thr(Bu$^t$)OBu$^t$.

**Revendications**

1. Procédé pour la préparation d'insuline humaine ou de ses dérivés à partir d'insuline de porc ou de ses dérivés, caractérisé en ce qu'on fait réagir l'insuline de porc ou un dérivé d'insuline de porc à une valeur de pH inférieure à son point isoélectrique, en présence de trypsine ou d'un ferment analogue à la trypsine, avec un excès d'un ester de thréonine ou d'un de ses dérivés avec un groupe amino libre et on élimine éventuellement ensuite le ou les groupe(s) protecteurs(s).

2. Procédé selon la revendication 1, caractérisé en ce qu'on effectue la réaction en l'absence d'un solvant organique, ein faisant réagir de préférece l'insuline de porc dans un milieu de réaction dont la teneur en eau est au moins égale ou supérieure à 50 volumes %.

3. Procédé selon l'une des revendications 1 et 2, caractérisé en ce que la réaction est effectuée en présence d'un acide organique faible.

4. Procédé selon l'une ou plusieurs des revendications 1 à 3, caractérisé en ce que la réaction est effectuée en présence d'acide acétique.

5. Procédé selon l'une ou plusieurs des revendications 1 à 4, caractérisé en ce qu'on travaille à une valeur de pH comprise entre 4 et le point isoélectrique.

6. Procédé selon la revendication 2, caractérisé en ce qu'on effectue la réaction en présence d'un acide organique faible, de préférence l'acide acétique.

7. Procédé selon l'une ou plusieurs des revendications 1 à 6, caractérisé en ce qu'on prépare la dés-Phe-$^{B1}$-insuline de porc.

8. Procédé selon l'une ou plusieurs des revendications 1 à 6, caractérisé en ce que le dérivé de l'insuline de porc est une insuline de porc qui porte un groupe protecteur en position N$^{\alpha B1}$.

9. Procédé selon la revendication 8, caractérisé en ce que le groupe protecteur est BOC ou DDZ.

10. Procédé selon l'une ou plusieurs des revendications 1 à 9, caractérisé en ce que la réaction est effectuée avec Thr(Bu$^t$)-OBu$^t$.